# EUROPEAN PATENT APPLICATION

(11) **EP 4 066 734 A1**
(43) Date of publication of application: **05.10.2022**
(21) Application number: 20893237.6
(22) Date of filing: 25.11.2020
(51) Int. Cl.: A61B 5/0205

(54) **METHOD AND APPARATUS FOR PROVIDING INFORMATION FOR RESPIRATORY TRAINING, ELECTRONIC DEVICE, SYSTEM, AND STORAGE MEDIUM**

(30) Priority: 25.11.2019 CN 201911165114
(71) Applicant: BOE Technology Group Co., Ltd., Beijing 100015 (CN)
(72) Inventor: HU, Yanyang, Beijing 100176 (CN); WANG, Hongliang, Beijing 100176 (CN); ZHANG, Xu, Beijing 100176 (CN); JIANG, Lan, Beijing 100176 (CN)
(74) Representative: Potter Clarkson
(86) International application number: PCT/CN2020/131494
(87) International publication number: WO 2021/104306

(57) **Abstract**

The present disclosure relates a method, device, electronic apparatus and system of providing information for breathing training, and storage medium. The method of providing information for breathing training includes: receiving first data input on a first display interface, the first data including breathing training time data; and presenting output data on a second display interface, the output data including breathing beat image prompting an exhaling action and an inhaling action by using alternate graphics.

## Description

### CROSS-REFERENCE TO RELATED APPLICATIONS

The present application claims priority to the Chinese Patent Application No. 201911165114.0, titled "METHOD, DEVICE, ELECTRONIC APPARATUS AND SYSTEM OF PROVIDING INFORMATION FOR BREATHING TRAINING, AND STORAGE MEDIUM" filed on November 25, 2019, the entire contents of which are incorporated herein by reference.

### TECHNICAL FIELD

The present disclosure relates to the field of personal health management, in particular, to a method, device, electronic apparatus and system of providing information for breathing training, and storage medium.

### BACKGROUND

With the improvement of living standard and the acceleration of social rhythm, people are paying more and more attention to personal health, and also maintain their personal health in a variety of ways, in which the easiest way is deep breathing. The deep breathing can expel residual gas and other metabolites in the lungs, and inhale more fresh air to supply the oxygen required by various organs and improve organ functions. The deep breathing can make related muscles and organs in chest and abdomen move to a greater extent, and can inhale more oxygen and exhale carbon dioxide, so that blood circulation can be strengthened.

Moreover, studies have shown that the deep breathing has a significant effect on the control of high blood pressure. The high blood pressure is a very common chronic disease. Excessive stress and mental stress are one of the main reasons leading to increased blood pressure. Therefore, physical and mental regulation has become the most basic preventive and treatment method of the high blood pressure. Stable and slow breathing training is an effective way to relax the body and mind and relieve tension. Studies have shown that after a few minutes of breathing training, muscles around blood vessels begin to relax, blood flow resistance decreases, and the blood pressure decreases significantly. By carrying on 4 to 6 weeks of regular breathing training with at least 10 minutes of effective breathing each time and at least 40 minutes of cumulative effective breathing time per week, the blood pressure can drop continuously and significantly, and the effect is stable and not easy to rebound.

But many people do not take deep breathing. Because the breathing is too short to allow air to enter into the lower end of the lung lobes, the ventilation volume is small, and only 1/3 of the lung is used in a lifetime. A British study showed that more than 90% of adults do not consciously regulate their breathing. In China, more than half of the people in cities breathe incorrectly. The short breathing not only leads to oxygen deficit of brains and fatiguability of many people, but also easily induces many diseases. In addition, effective deep breathing requires a uniform frequency, but it is difficult for a person to grasp the frequency of deep breathing.

Currently, there is no very simple and effective breathing training system and device on the market.

### SUMMARY

The present disclosure provides a method, device, electronic apparatus and system of providing information for breathing training, and storage medium.

A first aspect of the present disclosure provides a method of providing information for breathing training, including:
receiving first data input on a first display interface, the first data including breathing training time data; and
presenting, based on the first data, a breathing beat image on a second display interface, the breathing beat image prompting an exhaling action and an inhaling action by using alternate graphics.

In an embodiment, the second display interface also presents an ending icon, when the second display interface receives a pressing operation on the ending icon for a predetermined time, the second display interface stops presenting the breathing beat image, and the second display interface prompts that training data is not recorded when the breathing training time has not reached an effective training time.

In an embodiment, when the second display interface presents the breathing beat image, and receives an operation of a user on an other icon than the ending icon, a corresponding operation is not implemented.

In an embodiment, the second display interface also presents a sound icon, and when the second display interface receives an operation on the sound icon, sound is played or stopped, wherein the sound includes breathing beat sound, and the breathing beat sound prompts the exhaling action and the inhaling action by using different sounds.

In an embodiment, the first data further includes at least one of a historical record retrieving command and a help command.

In an embodiment, the sound further includes voice guidance, and the voice guidance is configured to prompt, by voice, a user to perform a corresponding action.

In an embodiment, the second display interface also presents a time display and/or a background display, the time display displays a training time of a user or a remaining time by counting up or down, and the background display is configured to adjust a light intensity, color or image of a background.

In an embodiment, the method further includes: presenting constructing training environment data on a third display interface to prompt a user of a training environment.

In an embodiment, the method further includes: receiving pre-training blood pressure data on a fourth display interface; and receiving post-training blood pressure data on a fifth display interface, wherein the blood pressure data is input manually or automatically by a detection device.

In an embodiment, in the case where the blood pressure data is input automatically by the detection device, the fourth display interface and the fifth display interface present a searching icon for searching the detection device, so that when the fourth display interface and the fifth display interface receive an operation on the searching icon, the detection device is searched.

In an embodiment, the method further includes: generating an evaluation report based on historical record data; and presenting content of the evaluation report on a sixth display interface.

In an embodiment, the method further includes: receiving breathing detection data when the second display interface is presented; comparing the breathing detection data with data of the breathing beat image; and presenting a reminder or waring to a user when there is a deviation between the breathing detection data and the data of the breathing beat image or the deviation reaches a threshold.

In an embodiment, the breathing training time data includes time length data of each breath and time length data of the breathing training.

In an embodiment, a time length of each beat of the breathing beat image is presented by a bubble icon, and the first display interface presents a plurality of bubble icons which are slidable, and the plurality of bubble icons present different time lengths for a user to choose.

In an embodiment, the time length data of the breathing training is presented on at least one of the first display interface and the second display interface through a time bar icon, and the time bar icon dynamically presents the time length of the breathing training and also presents an effective time length and an optimal time length of the breathing training.

In an embodiment, the method further includes, before receiving the first data input on the first display interface: receiving user identity data which is input, and determining a user identity according to the user identity data.

A second aspect of the present disclosure provides a device of providing information for breathing training, including a processor, a transceiver and a display, wherein the processor is configured to: control the transceiver to receive first data input on a first display interface, the first data including breathing training time data; and control the display to present, based on the first data, a breathing beat image on a second display interface, the breathing beat image prompting an exhaling action and an inhaling action by using alternate graphics.

In an embodiment, the processor is further configured to control the display to: also present an ending icon on the second display interface; and when the second display interface receives a pressing operation on the ending icon for a predetermined time, stop presenting the breathing beat image, and prompt that training data is not recorded when the breathing training time has not reached an effective training time.

In an embodiment, the processor is further configured to control the display not to implement a corresponding operation, when the second display interface presents the breathing beat image, and receives an operation of a user on an other icon than the ending icon.

In an embodiment, the processor is configured to: control the display to also present a sound icon on the second display interface; and play or stop sound when the second display interface receives an operation on the sound icon, wherein the sound includes breathing beat sound, and the breathing beat sound prompts the exhaling action and the inhaling action by using different sounds.

In an embodiment, the first data further includes at least one of a historical record retrieving command and a help command.

In an embodiment, the sound further includes voice guidance, and the voice guidance is configured to prompt, by voice, a user to perform a corresponding action.

In an embodiment, the processor is configured to control the display to also present a time display and/or a background display on the second display interface, the time display displays a training time of a user or a remaining time by counting up or down, and the background display can adjust a light intensity, color or image of a background.

In an embodiment, the processor is configured to: present constructing training environment data on a third display interface to prompt a user of a training environment.

In an embodiment, the processor is configured to control the transceiver to receive pre-training blood pressure data input on a fourth display interface, and receive post-training blood pressure data input on a fifth display interface, wherein the blood pressure data is input manually or automatically by a detection device.

In an embodiment, in the case where the blood pressure data is input automatically by the detection device, the fourth display interface and the fifth display interface present a searching icon for searching the detection device, so that when the fourth display interface and the fifth display interface receive an operation on the searching icon, the detection device is searched.

In an embodiment, the processor is configured to: generate an evaluation report based on historical record data; and control the display to present content of the evaluation report on a sixth display interface.

In an embodiment, the processor is configured to: control the transceiver to receive breathing detection data when controlling the display to present the second display interface; compare the breathing detection data with data of the breathing beat image; and present a reminder or waring to a user when there is a deviation between the breathing detection data and the data of the breathing beat image or the deviation reaches a threshold.

In an embodiment, the breathing training time data includes at least one of time length data of each breath and time length data of the breathing training.

In an embodiment, a time length of each beat of the breathing beat image is presented by a bubble icon, and the first display interface presents a plurality of bubble icons which are slidable, and the plurality of bubble icons present different time lengths for a user to choose.

In an embodiment, the time length data of the breathing training is presented on at least one of the first display interface and the second display interface through a time bar icon, and the time bar icon dynamically presents the time length of the breathing training and presents an effective time length and an optimal time length of the breathing training.

In an embodiment, the processor is configured to control the transceiver to receive user identity data which is input, and determine a user identity according to the user identity data before controlling the transceiver to receive the first data input on the first display interface.

A third aspect of the present disclosure provides a device of providing information for breathing training, including a memory, a processor, and a computer program stored in the memory and being executable on the processor, wherein the processor executes the computer program to implement the method of providing information for breathing training according to any one of embodiments in the first aspect.

A fourth aspect of the present disclosure provides a system of providing information for breathing training, including the device for providing information for breathing training according to the third aspect and a sphygmomanometer, wherein the sphygmomanometer is configured to provide blood pressure data to the device for providing information for breathing training.

In an embodiment, the system further includes: a breathing detection device, configured to detect breathing data of a user and provide the breathing data to the device for providing information for breathing training.

A fourth aspect of the present disclosure provides a computer-readable storage medium having a computer program stored thereon that, when being executed by a processor, implements the method of providing information for breathing training according to any one of embodiments in the first aspect.

### BRIEF DESCRIPTION OF THE DRAWINGS

FIG. 1 is a schematic diagram of a method of providing information for breathing training according to an embodiment of the present disclosure;
FIG. 2 is a schematic diagram of a method for providing information for breathing training according to another embodiment of the present disclosure;
FIG. 3 is a flowchart of a method flow of providing information for breathing training according to an embodiment of the present disclosure;
FIG. 4 is a view of a first step in FIG. 3 according to an embodiment of the present disclosure;
FIG. 5 is a view of a second step in FIG. 3 according to an embodiment of the present disclosure;
FIG. 6 is a view of a third step in FIG. 3 according to an embodiment of the present disclosure;
FIG. 7 is a view of a fourth step in FIG. 3 according to an embodiment of the present disclosure;
FIG. 8 is a view of a fifth step in FIG. 3 according to an embodiment of the present disclosure;
FIG. 9 is a view of a sixth step in FIG. 3 according to an embodiment of the present disclosure;
FIG. 10 is a structural diagram of a device of providing information for breathing training according to an embodiment of the present disclosure;
FIG. 11 is a structural diagram of an electronic apparatus according to an embodiment of the present disclosure; and
FIG. 12 is a structural diagram of a device of providing information for breathing training according to another embodiment of the present disclosure.

### DETAILED DESCRIPTION

In order to enable those skilled in the art to better understand the present disclosure, the present disclosure will be further described in detail below with reference to the accompanying drawings and implementations. It shall be noted that the embodiments and the features in the embodiments of the present application can be combined with each other arbitrarily in case where there is no conflict.

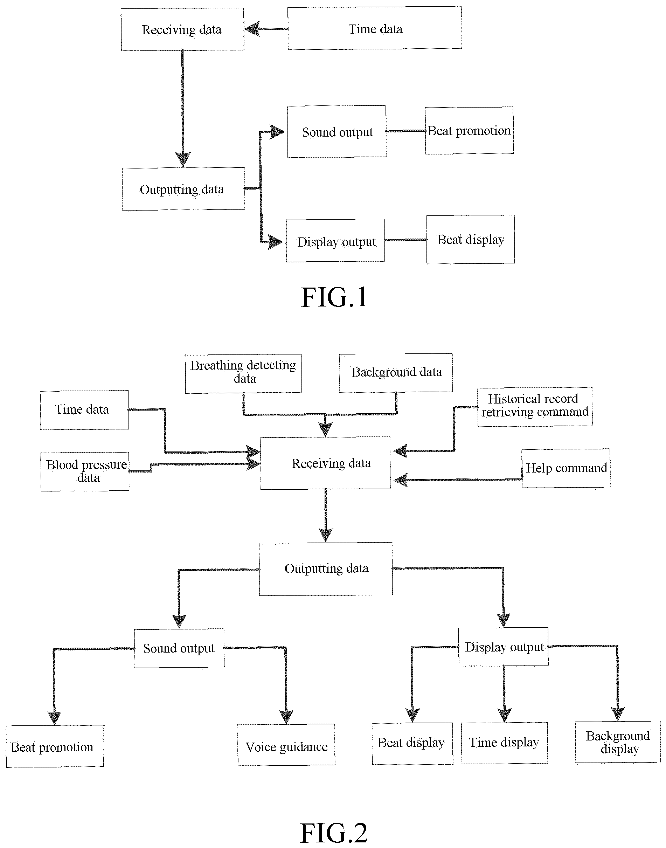

As shown in FIG. 1, a method of providing information for breathing training in an embodiment of the present disclosure includes:
1) receiving first data input on a first display interface, the first data including breathing training time data; and
2) presenting output data on a second display interface, the output data including breathing beat image and the breathing beat image prompting an exhaling action and an inhaling action by using alternate graphics.

In the method of providing information for breathing training according to the embodiment of the present disclosure, the breathing beat image is presented on the display interface to guide the user to perform breathing training, so the method is simple and convenient.

In addition, according to the embodiment of the present disclosure, a plurality of display interfaces including the first display interface and the second display interface are provided, which facilitates the clear presentation of content to the user and facilitates the operation of the user.

According to an embodiment of the present disclosure, the first display interface and the second display interface may be different interfaces that are not displayed at the same time. However, the present disclosure is not limited thereto, and the first display interface and the second display interface may be located on the same display interface and displayed at the same time, but are displayed on different parts of the same display interface.

The breathing training time data includes time length data of each breath, such as 6 seconds, 7.5 seconds, 9 seconds and the like. For example, the breathing training time data includes total time length data of the breathing training, such as 10 minutes, 15 minutes, 20 minutes, 25 minutes, unlimited time and the like, to limit the training time. The time length of each beat of the beat sound and beat image is determined based on the time length of each breath. Based on the total time length data of the breathing training, the total time length of the beat sound and beat image is determined. In another implementation, the total time length data of the breathing training is not input, but is set by default, such as 30 minutes, and the progress of the training time length is displayed in real time on the second display interface.

The training breathing time data may be directly input, for example, a specific time value is input. Optionally, the training breathing time data may be a selection from a plurality of recommended schemes of breathing time data, for example, a plurality of times are displayed on the interface or upper and lower limits of time is displayed in a sliding or scrolling manner, from which the training breathing time data is selected.

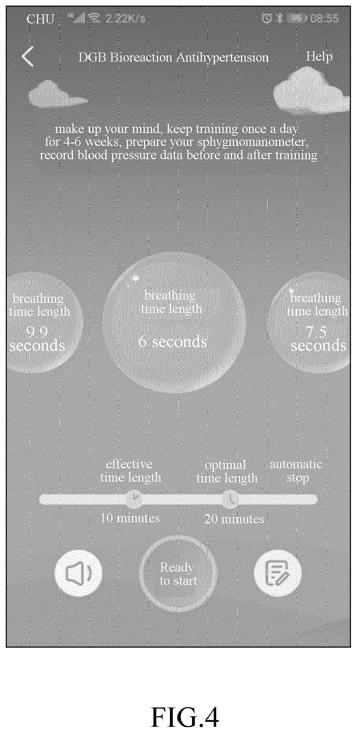

For example, as shown in FIG. 4, the time length of each beat of the breathing beat image may be presented by a bubble icon. A plurality of slidable bubble icons are presented on the first display interface, and the plurality of bubble icons are presented with different time lengths for a user to choose. For example, different bubble icons are presented 6 seconds, 7.5 seconds, and 9 seconds respectively, and the bubble icons may be slid for the user to choose, in which the currently selected time length is displayed in an enlarged manner. Here, the time length of each beat presented by the bubble icon for the user to choose may be the time length determined by experiments through which time length of the breathing training blood pressure can be effectively reduced.

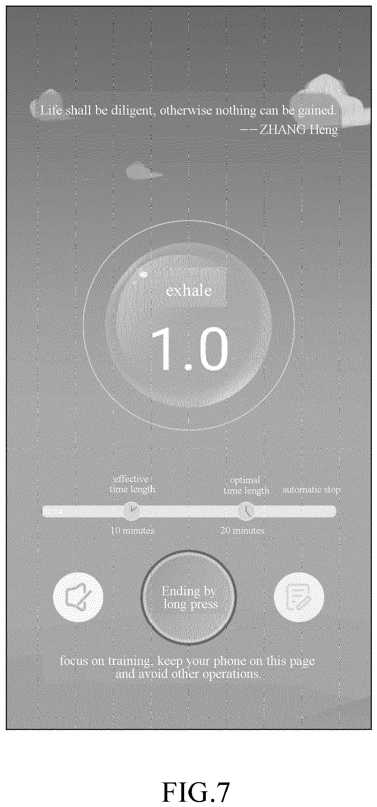

In addition, as shown in FIGS. 4 and 7, the time length data of the breathing training may be presented on the first display interface and the second display interface through a time bar icon. The time bar icon may dynamically present the time length of the breathing training and present an effective time length, optimal time length and automatic stop time of the breathing training. In this way, it is beneficial to intuitively present the time length of the breathing training to the user, and prompt the user to perform the effective time length and optimal time length of the breathing training. The effective time length means that the breathing training with a time length being longer than the effective time length is effective, and 10 minutes is shown in the drawings as an example. The optimal time length means an optimal time length of the breathing training, and 20 minutes is shown in the drawings as an example. The automatic stop means that the process will be stopped automatically after it is over a specified time, for example 30 minutes. The training longer than such specified time is not recommended, and it may happen that the user falls asleep.

The display output may be a reminder of a breathing character, or a reminder of a breathing action image. For example, the interface displays "exhale" or "exhalation" during exhalation, and "inhale" or "inhalation" during inhalation. For another example, the interface displays an exhaling image during exhalation, and the interface displays an inhaling image during inhalation, for example, through a facial shape change of a cartoon character, or a graphic display of gas direction. In an optional solution, while the breathing action is promoted, the display output further includes a time change, for example, countdown for each breath, promoting of the number of minutes of the training conducting time through countup or countdown, or promoting of the change of the training time through a progress bar or a combination of the progress bar and time, as shown in FIG. 7.

According to an embodiment, as shown in FIG. 7, an ending icon (i.e., the icon "long press for ending") is also presented on the second display interface. When the second display interface receives a pressing operation on the ending icon for a predetermined time, the second display interface stops presenting the breathing beat image, and the second display interface prompts that training data is not recorded when the breathing training time has not reached an effective training time. The predetermined time may be greater than or equal to 3 seconds. However, the present disclosure is not limited thereto, and for example, the predetermined time may be greater than or equal to 1 second or 2 seconds, greater than or equal to 4 seconds, 5 seconds, 7 seconds, and the like.

According to an embodiment, as shown in FIG. 7, when the second display interface presents the breathing beat image, and receives an operation of a user on an other icon than the ending icon, a corresponding operation can not be implemented, which is helpful for the user to train more attentively.

According to an embodiment, the second display interface also presents a sound icon, and when the second display interface receives an operation on the sound icon, sound is played or stopped. The sound includes breathing beat sound, the breathing beat sound prompts the exhaling action and the inhaling action by using different sounds, and FIG. 4 shows a loudspeaker icon as the sound icon.

The breathing beat sound may be for example a pronunciation of "exhale" and "inhale", a sound that imitates breathing, or other sounds that allow the user to distinguish between exhalation and inhalation. For example, at fixed time intervals, such as every minute, a special sound may be issued to promote the user, such as telling the time in minutes, and the like (the sound may be so small that it does not affect the breathing beat sound).

For example, when the display output and the voice output exist at the same time, the breathing beat sound and the beat of the beat image are synchronized.

With the above technical solution, the user may perform breathing training according to the beat provided by the beat image and/or the beat sound, and can determine the breathing training mode by himself, and the beat image can help the user precisely control the beat and time length of the breathing, so that the breathing training is more effective. In addition, the user may accurately control the beat and time length of breathing with the promotion of auxiliary voice even without looking at the image.

In another solution, the method further includes presenting constructing training environment data on a third display interface for prompting a user to find the elements that can achieve the optimal training effect, and the elements include data such as room, body position, relaxation, breathing movement and the like, for example, "a quiet room", "the best position is to sit on a chair aslant ", "breathe calmly for a minute", "take abdominal breathing" and the like.

In another solution, as shown in FIG. 2, the first data further includes at least one of a historical record retrieving command or a help command.

The historical record retrieving command is used to retrieve historical record data of the user to be output in a voice and/or display mode. Optionally, the training effect of the user may also be evaluated in combination with the historical record data, and then a evaluation report is generated. The evaluation report may be provided to the user in a voice and/or display mode. Furthermore, a next training suggestion may be provided to the user in a display and/or voice mode. With such solution, the previous training effect of the user may be easily evaluated, and a guidance may be provided for the user's next training.

The help command is used to provide training-related help to the user, such as text, voice, and video help, including training action guidance voice, video, note, text and voice of related content, and the like. With such solution, the user may get help they want in time before training, for example, interface operation guidance, breathing action example and the like, which may avoid wrong training or improve training efficiency and effectiveness.

At the same time, the method further includes presenting training effect evaluation data on a sixth display interface, and the effect evaluation data may be presented in the form of an evaluation report. The method further includes presenting help content data on a seventh display interface. There is no specific sequence among the second display interface, the sixth display interface, and the seventh display interface.

According to an embodiment, as shown in FIG. 2, the method further includes receiving pre-training blood pressure data on a fourth display interface and receiving post-training blood pressure data on a fifth display interface. The pre-training blood pressure data is the data of the user measured before the breathing training, and the data may be input manually or automatically by a detection device (for example, a blood pressure meter and the like). The post-training blood pressure data is the data of the user measured after the breathing training, and the data may be input manually or automatically by the detection device. The fourth display interface is presented before the breathing training of the second display interface starts, and the fifth display interface is presented after the breathing training of the second display interface is completed. By comparing the pre-training blood pressure data and the post-training blood pressure data, the effect of the current breathing training and the historical training effect can be comprehensively evaluated.

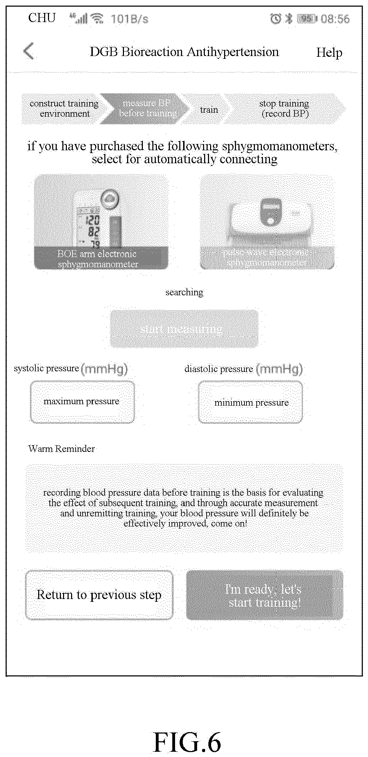

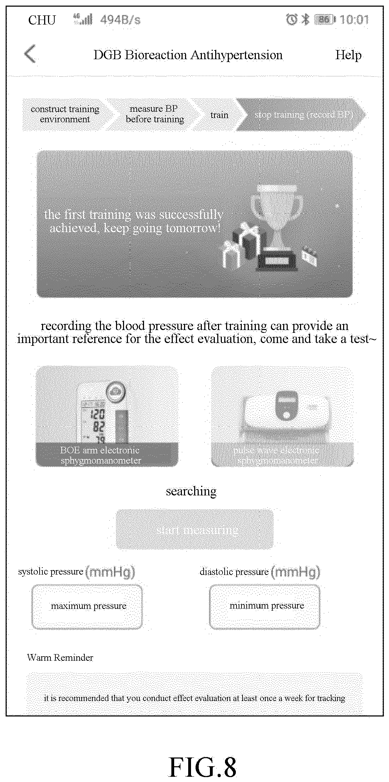

According to an embodiment, as shown in FIGS. 6 and 8, in the case where the blood pressure data is input automatically by the detection device, the fourth display interface and the fifth display interface present a searching icon for searching the detection device, so that when the fourth display interface and the fifth display interface receive an operation on the searching icon, the detection device is searched.

According to an embodiment, the method further includes: determining, according to user identity data which is input, a user identity, before receiving the first data input on the first display interface. The user identity data includes but is not limited to password data, fingerprint data, face identification data and the like. The method further includes: after determining the user identity, reading data corresponding to the user identity, such as default breathing training time data, historical record data, and the like. The default breathing training time data may be time data initially determined according to the rules of most people, or may be determined according to the time data of the last training of the user.

According to an embodiment, the first interface also presents a sound switch for turning on or off a sound, and the sound may be a background sound of the interface, a prompt sound to the user or the like.

According to an embodiment, the method further includes receiving input background data at the same time as or before the presentation of the second display interface. The background data may be artificially set, for example, a selection of a day mode, night mode, background color of different brightness, and a setting of a background pattern and the like. The background data may also be automatically input according to a detected ambient light factor, that is, automatically adjusted.

According to an embodiment, the method further includes receiving breathing detection data when the second display interface is presented. For example, when the user is performing breathing training, the breathing detection data of the user such as an exhaling time length of each breathing beat, an inhaling time length of each breathing beat, and a total time length of each breathing beat are received from the user. According to an embodiment of the present disclosure, the breathing detection data may be detected and input by, for example, a breathing sensor worn on the chest of the user that can detect the above breathing detection data. According to an embodiment of the present disclosure, the method further includes comparing the breathing detection data with the output data presented on the interface, and presenting a reminder or waring to a user when there is a deviation therebetween or the deviation reaches a threshold. For example, the exhaling time length of each breathing beat, the inhaling time length of each breathing beat, and/or the total time length of each breathing beat of the user detected by the breathing sensor may be respectively compared with the exhaling time length, the inhaling time length, and/or the exhaling and inhaling total time length of the breathing beat image presented on the second display interface, and a reminder or waring is presented to the user when there is a deviation therebetween or the deviation reaches a threshold. With such solution, it can monitor in real time whether the user is training according to a correct training method, and if not, the user is promptly prompted for correction to ensure the training effect.

A plurality of interfaces are described above for receiving various data inputs. However, it shall be understood that some inputs may be received on one interface or part of the interface content may be divided into multiple interfaces for presentation. For example, the pre-training blood pressure data may be presented on the first display interface, and the help command and historical record retrieving command may be presented on other interfaces separately.

According to an embodiment, the sound output further includes voice guidance, and the voice guidance is used to prompt, by voice, a user to perform a corresponding action such as body position, action, some notes and the like. The voice guidance may also provide encouraging words for the user to increase the persistence time length of the user.

The display output also includes a time display and/or background display. The time display may display a training time of a user or a remaining time by counting up or down. The background display may adjust, according to a received input, a light intensity of background, for example a day mode and a night mode. The background display may also adjust color or image of the background to be suitable for different people.

For example, the method further includes storing data into a memory and/or retrieving data from the memory. For example, the data of each training is stored in a historical record database, and when the historical data needs to be viewed, the historical data is retrieved from the database. For another example, video, text, voice and other data related to the help command are also stored in the database, and can be retrieved and output as needed.

According to an embodiment, the method further includes presenting a training reminder to the user. The training reminder may be presented when the homepage is entered, or may be presented in the form of an application notification after being generated in the background. The form of the reminder may be a pop-up window, a banner, a text message and the like.

In addition, the evaluation report may also be shared on the sixth interface, for example, through WeChat, Weibo, or other applications to share with relatives and friends.

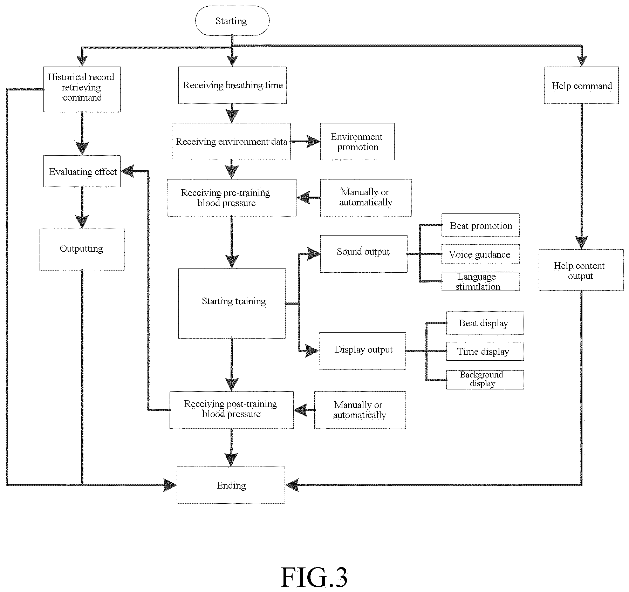

A specific embodiment of the present disclosure will be described below with reference to FIGS. 3 to 9.

1) After the homepage is entered, the breathing time data or historical record retrieving command or help command (referring to the interface in FIG. 4, which corresponds to the first display interface) may be selected to be received. The total time length of breathing training and the time length of each breathing may be received on the current interface. There are the time lengths of each breathing of 9.9 seconds, 7.5 seconds, and 6 seconds for selection. The current selection is 6 seconds for each breathing, and 30 minutes for the total training time length, and the interface also prompts that the effective time length is more than 10 minutes, 20 minutes is the optimal time length, and it will automatically stop at 30 minutes.

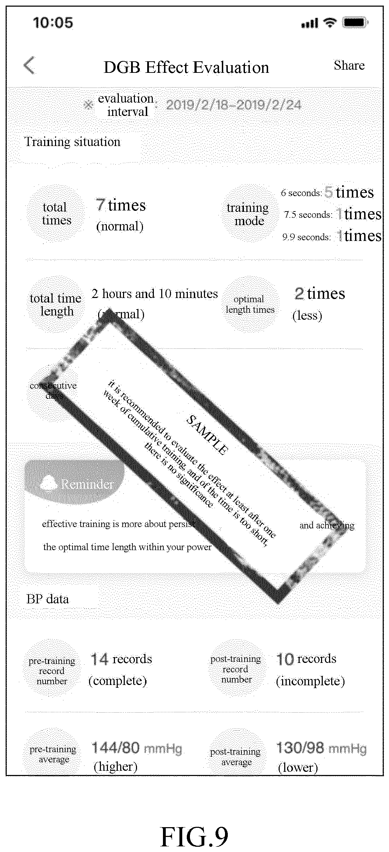

2) If the received command is a historical record retrieving command, the historical record data may be output in a sound and/or display form after being processed, and then it is ended. Optionally, the historical record data may also be analyzed and evaluated effectively to generate an evaluation report, and then is output in a sound and/or display form (referring to the interface in FIG. 9, which corresponds to the sixth display interface), and then the operation is ended or returned to the beginning steps. FIG. 9 exemplarily shows that the report includes the number of times of the training that have been performed, the number of the training in various training modes (different breathing time lengths), the total training time length, the number of times of the training reaching the optimal time length (for example, reaching 20 minutes and longer), the blood pressure data and the like, and then according to preset data, it is determined whether the above data is normal, such as high blood pressure, low frequency, and the like. In addition, in an optional solution, a recommended training scheme for the next step may be given based on the above-mentioned data.

If the help command is received, the related help content will be output in a sound and/or display form, and then the operation is ended or returned to the beginning step.

If the breathing time data is received, it goes to step 3).

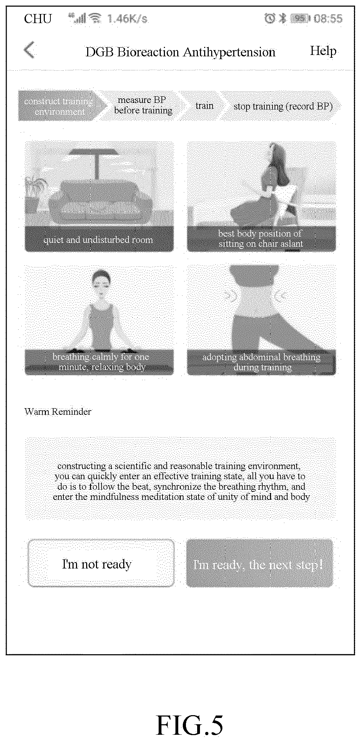

3) The training environment data is constructed and presented (referring to the interface in FIG. 5, which corresponds to the third display interface). At this time, the example shown in the drawings prompts "quiet and undisturbed room", "best body position of sitting on chair aslant", "breathing calmly for one minute", "adopting abdominal breathing". When it is ready, it may proceed to the next step or return to the previous step.

4) Optionally, the pre-training blood pressure data is received. The pre-training blood pressure data may be input manually or automatically by a sphygmomanometer (referring to the interface in FIG. 6, which corresponds to the fourth display interface). Different sphygmomanometers (such as Bluetooth sphygmomanometer) may be selected on the interface, and then the detection is controlled to start and obtain relevant data. Optionally, the blood pressure data may be manually entered, and then it may choose to enter the next step or return to the previous step.

5) The breathing training starts to train the breathing time length and frequency of the user through sound beats and/or display beats, and use other sound/display means to stimulate and promote the user (referring to the interface in FIG. 7, which corresponds to the second display interface). In the drawings, a text is used to prompt exhaling and inhaling, and a countdown is used to display the current remaining time length of exhaling, and the training may be ended on the interface.

6) Optionally, the post-training blood pressure data after training may be received (referring to the interface in FIG. 8, which corresponds to the fifth display interface), and the interface presentation thereof is the same as in step 4. When it is ended, the record of the current training may be automatically stored. Optionally, an effect evaluation may be performed (referring to FIG. 9), and then the results may be output through display and/or voice, and then the operation is ended.

The inputs of the breathing time data, environment data, and pre-training blood pressure data is not limited to the above sequence, and the breathing time data, environment data, and pre-training blood pressure data may be input at the same time or be adjusted to be input in another sequence.

The present disclosure also provides a device 100 of providing information for breathing training, which includes:
1) a data receiving module 101, configured to receive first data input, the first data including breathing training time data; and
2) a data presenting module, configured to output data in a display manner, the output data including breathing beat image that prompts an exhaling action and an inhaling action by using alternate graphics.

For example, the data receiving module 101 is also configured to receive at least one or more of user identity data, background data, breathing detection data, pre-training blood pressure data, post-training blood pressure data, historical record retrieving command, help command, and the like, which may be input manually or automatically, for example, is input through the wireless transmission or is input automatically after the detection by a device. For example, the data receiving module 101 includes a detecting module, and the detecting module includes a user identity detecting module, an ambient light detecting module, a breathing detecting module, a blood pressure detecting module, and the like. The user identity detecting module is used to detect the user identity, for example, through fingerprint and facial data, the breathing detecting module is used to detect the actual breathing data of a user, the ambient light detecting module is used to detect the intensity of the ambient light, and the blood pressure detecting module is used to detect the blood pressure of a user.

For example, the data receiving module 101 further includes a processing module, which is used to process various received data and provide the processed data to the data presenting module 102. The processing module preferably further includes a storage module and a retrieving module, which are respectively used to store data in the database and retrieve data from the database.

The data presenting module 102 is used to output the processed data in a display manner. Optionally, at the same time as the display output, the sound output is also performed, which specifically includes at least one of breathing beat sound, voice guidance, and language stimulation. The voice guidance is used to prompt the user through voice to perform a corresponding action, such as body position, action, some notes and the like. The language stimulation is used to provide encouraging words to the user to improve the persistence time length of the user. The data presenting module 102 also presents at least one of time display and background display. The beat display may be a reminder of breathing characters, or a reminder of breathing action image. The time display may display a training time length of the user or a remaining time length by counting up or down. The background display may adjust the intensity of the background light according to the received input, such as day mode, night mode. The background display may also adjust color or image of the background to meet the needs of different people.

The present disclosure can also integrate the breathing training method in an App. The user opens a breathing and reducing blood pressure training application program, uses a play guiding video for the first time, and receives help after the play is completed, which can be watched in the help later. The App may be used in personal smart terminals, such as mobile phones, Pads, and computers, and may also be deployed in public health infrastructure as a kind of national health service.

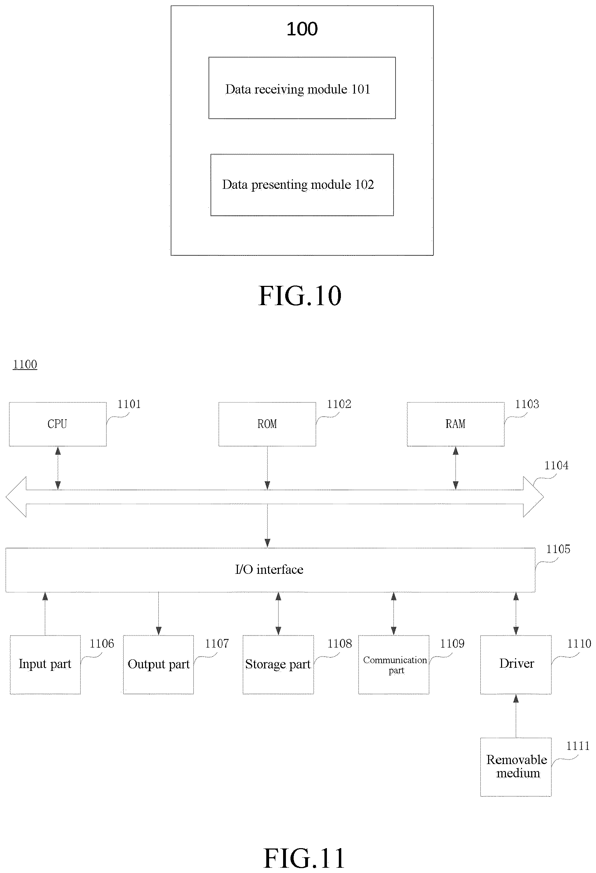

Next, referring to FIG. 11, a schematic structural diagram of an electronic apparatus 1100 suitable for implementing the device according to the embodiment of the present disclosure is shown.

As shown in FIG. 11, the electronic apparatus 1100 includes a central processing unit (CPU) 1101, which can perform various appropriate actions and processing based on a program stored in a read-only memory (ROM) 1102 or a program loaded from a storage part 1108 into a random access memory (RAM) 1103. The RAM 1103 therein further stores various programs and data required for the operation of the system 1100. The CPU 1101, ROM 1102, and RAM 1103 are connected to each other through a circuit 1104. An input/output (I/O) interface 1105 is also connected to the circuit 1104.

The following components are connected to the I/O interface 1105: an input part 1106 including a touch screen; an output part 1107 including for example a liquid crystal display panel (LCD) and a speaker; a storage part 1108 including a hard disk; and a communication part 1109 including a network interface such as an antenna, a modem and the like. The communication part 1109 performs communication processing via a network such as the Internet. A driver 1110 is also connected to the I/O interface 1105 as needed. A removable medium 1111 such as a magnetic disk, an optical disk, a magneto-optical disk, a semiconductor memory and the like is installed on the driver 1110 as needed, so that the computer program read therefrom is installed into the storage portion 1108 as needed.

In particular, according to an embodiment of the present disclosure, the methods and processes described above with reference to the flowcharts of FIGS. 1-9 may be implemented as computer software programs. For example, the present disclosure includes a computer program product, which includes a computer program carried on a machine-readable medium, and the computer program contains program code for executing the method shown in the flowchart. In such an embodiment, the computer program may be downloaded and installed from the network through the communication part 1109, and/or installed from the removable medium 1111. When the computer program is executed by the central processing unit (CPU) 1101, the above-mentioned functions defined in the system of the present disclosure are executed.

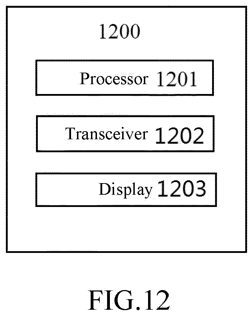

Referring to FIG. 12, the present disclosure further provides another device 1200 of providing information for breathing training, including a processor 1201, a transceiver 1202 and a display 1203. The processor 1201 is configured to: control the transceiver 1202 to receive first data input on a first display interface, the first data including breathing training time data; and control the display 1203 to present, based on the first data, a breathing beat image on a second display interface, the breathing beat image prompting an exhaling action and an inhaling action by using alternate graphics.

In an embodiment, the processor 1201 is further configured to control the display 1203 to: also present an ending icon on the second display interface; and when the second display interface receives a pressing operation on the ending icon for a predetermined time, stop presenting the breathing beat image, and prompt that training data is not recorded when the breathing training time has not reached an effective training time.

In an embodiment, the processor 1201 is further configured to control the display 1203 to further present reminding information on the second display interface to promote a user to remain on the second display interface and avoid other operation while performing the training.

In an embodiment, the processor 1201 is configured to: control the display 1203 to also present a sound icon on the second display interface; and play or stop sound when the second display interface receives an operation on the sound icon. The sound includes breathing beat sound, and the breathing beat sound prompts the exhaling action and the inhaling action by using different sounds.

In an embodiment, the first data further includes at least one of a historical record retrieving command and a help command.

In an embodiment, the sound further includes voice guidance, and the voice guidance is configured to prompt, by voice, a user to perform a corresponding action.

In an embodiment, the processor 1201 is configured to control the display 1203 to also present a time display and/or a background display on the second display interface, the time display displays a training time of a user or a remaining time by counting up or down, and the background display can adjust a light intensity, color or image of a background.

In an embodiment, the processor 1201 is configured to: present constructing training environment data on a third display interface to prompt a user of a training environment.

In an embodiment, the processor 1201 is configured to control the transceiver 1202 to receive pre-training blood pressure data input on a fourth display interface, and receive post-training blood pressure data input on a fifth display interface. The blood pressure data is input manually or automatically by a detection device.

In an embodiment, in the case where the blood pressure data is input automatically by the detection device, the fourth display interface and the fifth display interface present a searching icon for searching the detection device, so that when the fourth display interface and the fifth display interface receive an operation on the searching icon, the detection device is searched.

In an embodiment, the processor 1201 is configured to: generate an evaluation report based on historical record data; and control the display 1203 to present content of the evaluation report on a sixth display interface.

In an embodiment, the processor 1201 is configured to: control the transceiver 1202 to receive breathing detection data when controlling the display 1203 to present the second display interface; compare the breathing detection data with data of the breathing beat image; and present a reminder or waring to a user when there is a deviation between the breathing detection data and the data of the breathing beat image or the deviation reaches a threshold.

In an embodiment, the breathing training time data includes at least one of time length data of each breath and time length data of the breathing training.

In an embodiment, a time length of each beat of the breathing beat image is presented by a bubble icon, and the first display interface presents a plurality of bubble icons which are slidable, and the plurality of bubble icons present different time lengths for a user to choose.

In an embodiment, the time length data of the breathing training is presented on the second display interface through a time bar icon, and the time bar icon dynamically presents the time length of the breathing training and presents an effective time length and an optimal time length of the breathing training.

In an embodiment, the processor 1201 is configured to control the transceiver 1202 to receive user identity data which is input, and determine a user identity according to the user identity data before controlling the transceiver 1202 to receive the first data input on the first display interface.

The present disclosure also provides a system of providing information for breathing training, which includes the above-mentioned electronic apparatus, and also includes a sphygmomanometer. The sphygmomanometer is used to provide blood pressure data to the electronic apparatus, specifically including pre-training user blood pressure and post-training user blood pressure. According to an embodiment, the system further includes a breathing detection device for detecting breathing data of a user and providing the same to the electronic apparatus. The system may compare the detected breathing data with the data provided in the electronic apparatus to monitor whether the user trains in accordance with the prescribed actions. When there is a deviation in the breathing time length and beat, or the deviation reaches a certain threshold, a reminder or warning is issued to the user.

It shall be noted that the computer-readable medium shown in the present disclosure may be a computer-readable signal medium or a computer-readable storage medium, or any combination thereof. The computer-readable storage medium may be, for example, but not limited to an electrical, magnetic, optical, electromagnetic, infrared, or semiconductor system, device, or element, or any combination thereof. A specific example of the computer-readable storage media may include, but is not limited to: an electrical connection with one or more wires, portable computer disk, hard disk, random access memory (RAM), read-only memory (ROM), erasable removable programmable read-only memory (EPROM or flash memory), optical fiber, portable compact disk read-only memory (CD-ROM), optical storage device, magnetic storage device, or any suitable combination thereof. In the present disclosure, a computer-readable storage medium may be any tangible medium that contains or stores a program, and the program may be used by or in combination with an instruction execution system, apparatus, or device. In the present disclosure, the computer-readable signal medium may include a data signal propagated in a baseband or as a part of a carrier wave, in which a computer-readable program code is carried. The propagated data signal can take many forms, including but not limited to electromagnetic signal, optical signal, or any suitable combination thereof. The computer-readable signal medium may also be any computer-readable medium other than a computer-readable storage medium. The computer-readable medium may send, propagate, or transmit the program for use by or in combination with the instruction execution system, apparatus, or device. The program code contained on the computer-readable medium can be transmitted by any suitable medium, including but not limited to: wireless, wire, optical cable, RF and the like, or any suitable combination thereof.

The flowcharts and block diagrams in the accompanying drawings illustrate a possible implementation architecture, function, and operation of the system, method, and computer program product according to various embodiments of the present disclosure. In this regard, each block in the flowchart or block diagram may represent a module, program segment, or part of the code, and the aforementioned module, program segment, or part of the code contains one or more executable instructions for realizing a specified logical function. It shall also be noted that in some alternative implementations, the functions marked in the block may also occur in a different order from the order marked in the drawings. For example, two blocks shown in succession can actually be executed substantially in parallel, and they can sometimes be executed in a reverse order, depending on the functions involved. It shall also be noted that each block in the block diagram and/or flowchart, and the combination of the blocks in the block diagram and/or flowchart, can be implemented by a dedicated hardware-based system that performs the specified functions or operations or can be realized by a combination of dedicated hardware and computer instruction.

The unit or module involved in the embodiments described in the present disclosure may be implemented in software or hardware. The described unit or module may also be provided in the processor, for example, it may be described as: a processor includes a data receiving module 101 and a data presenting module 102. The names of these units or modules do not constitute a limitation on the units or modules themselves under certain circumstances. For example, the data receiving module 101 may also be described as "module for receiving data".

As another aspect, the present disclosure also provides a computer-readable storage medium. The computer-readable storage medium may be included in the electronic apparatus described in the above-mentioned embodiments; and may also exist alone without being assembled into the electronic apparatus. The foregoing computer-readable storage medium stores one or more programs, and when the foregoing programs are executed by one or more processors, the configuration method described in the present disclosure is implemented.

With the solution of the present disclosure, it may be convenient for a user to perform breathing training, and the frequency and time length of breathing may be accurately determined through the beat image and/or beat sound, which greatly improves the experience and training effect of the user. Moreover, by introducing blood pressure data before and after training, the training effect may be visually presented. In addition, the evaluation report may be generated by retrieving the historical records to fully show the phased training effect of the user and provide suggestions for the next training. In addition, by introducing the breathing detecting device, the actual breathing data of the user can be compared with the breathing data displayed on the interface to determine whether the user is training according to the promotion, and the user may be promoted to improve the training effect.

The above description is only a preferred embodiment of the present application and an explanation of the applied technical principles. Those skilled in the art shall understand that the scope of the present disclosure is not limited to the technical solutions formed by the specific combination of the above technical features, and shall also cover the technical solutions formed by any combination of the above technical features or equivalents thereof without departing from the foregoing disclosed concept, for example, a technical solution formed by mutually replacing the above features and the technical features disclosed (but not limited to) in the present application with similar functions.

## Claims

1. A method of providing information for breathing training, comprising:
receiving first data input on a first display interface, the first data comprising breathing training time data; and
presenting, based on the first data, a breathing beat image on a second display interface, the breathing beat image prompting an exhaling action and an inhaling action by using alternate graphics.

2. The method according to claim 1, wherein the second display interface also presents an ending icon,
when the second display interface receives a pressing operation on the ending icon for a predetermined time, the second display interface stops presenting the breathing beat image, and the second display interface prompts that training data is not recorded when the breathing training time has not reached an effective training time.

3. The method according to claim 2, wherein when the second display interface presents the breathing beat image, and receives an operation of a user on an other icon than the ending icon, a corresponding operation is not implemented.

4. The method according to any one of claims 1 to 3, wherein the second display interface also presents a sound icon, and
when the second display interface receives an operation on the sound icon, sound is played or stopped,
wherein the sound comprises breathing beat sound, and the breathing beat sound prompts the exhaling action and the inhaling action by using different sounds.

5. The method according to any one of claims 1 to 4, wherein the first data further comprises at least one of a historical record retrieving command and a help command.

6. The method according to claim 4, wherein the sound further comprises voice guidance, and the voice guidance is configured to prompt, by voice, a user to perform a corresponding action.

7. The method according to any one of claims 1 to 6, wherein the second display interface also presents a time display and/or a background display, the time display displays a training time of a user or a remaining time by counting up or down, and the background display is configured to adjust a light intensity, color or image of a background.

8. The method according to any one of claims 1 to 7, further comprising:
presenting constructing training environment data on a third display interface to prompt a user of a training environment.

9. The method according to any one of claims 1 to 8, further comprising:
receiving pre-training blood pressure data on a fourth display interface; and
receiving post-training blood pressure data on a fifth display interface,
wherein the blood pressure data is input manually or automatically by a detection device.

10. The method according to claim 9, wherein in the case where the blood pressure data is input automatically by the detection device, the fourth display interface and the fifth display interface present a searching icon for searching the detection device, so that when the fourth display interface and the fifth display interface receive an operation on the searching icon, the detection device is searched.

11. The method according to any one of claims 1 to 10, further comprising:
generating an evaluation report based on historical record data; and
presenting content of the evaluation report on a sixth display interface.

12. The method according to any one of claims 1 to 11, further comprising:
receiving breathing detection data when the second display interface is presented;
comparing the breathing detection data with data of the breathing beat image; and
presenting a reminder or waring to a user when there is a deviation between the breathing detection data and the data of the breathing beat image or the deviation reaches a threshold.

13. The method according to any one of claims 1 to 12, wherein the breathing training time data comprises time length data of each breath and time length data of the breathing training.

14. The method according to claim 13, wherein a time length of each beat of the breathing beat image is presented by a bubble icon, and
the first display interface presents a plurality of bubble icons which are slidable, and the plurality of bubble icons present different time lengths for a user to choose.

15. The method according to claim 13 or 14, wherein the time length data of the breathing training is presented on at least one of the first display interface and the second display interface through a time bar icon, and the time bar icon dynamically presents the time length of the breathing training and also presents an effective time length and an optimal time length of the breathing training.

16. The method according to any one of claims 1 to 15, further comprising, before receiving the first data input on the first display interface:
receiving user identity data which is input, and determining a user identity according to the user identity data.

17. A device of providing information for breathing training, comprising a processor, a transceiver and a display,
wherein the processor is configured to:
control the transceiver to receive first data input on a first display interface, the first data comprising breathing training time data; and
control the display to present, based on the first data, a breathing beat image on a second display interface, the breathing beat image prompting an exhaling action and an inhaling action by using alternate graphics.

18. The device according to claim 17, wherein the processor is further configured to control the display to:
also present an ending icon on the second display interface; and
when the second display interface receives a pressing operation on the ending icon for a predetermined time, stop presenting the breathing beat image, and prompt that training data is not recorded when the breathing training time has not reached an effective training time.

19. The device according to claim 18, wherein the processor is further configured to control the display not to implement a corresponding operation, when the second display interface presents the breathing beat image, and receives an operation of a user on an other icon than the ending icon.

20. The device according to any one of claims 17 to 19, wherein the processor is configured to:
control the display to also present a sound icon on the second display interface; and
play or stop sound when the second display interface receives an operation on the sound icon,
wherein the sound comprises breathing beat sound, and the breathing beat sound prompts the exhaling action and the inhaling action by using different sounds.

21. The device according to any one of claims 17 to 20, wherein the processor is configured to:
present constructing training environment data on a third display interface to prompt a user of a training environment.

22. The device according to any one of claims 17 to 21, wherein the processor is configured to control the transceiver to receive pre-training blood pressure data input on a fourth display interface, and receive post-training blood pressure data input on a fifth display interface,
wherein the blood pressure data is input manually or automatically by a detection device.

23. The device according to claim 22, wherein in the case where the blood pressure data is input automatically by the detection device, the fourth display interface and the fifth display interface present a searching icon for searching the detection device, so that when the fourth display interface and the fifth display interface receive an operation on the searching icon, the detection device is searched.

24. The device according to any one of claims 17 to 23, wherein the processor is configured to:
generate an evaluation report based on historical record data; and
control the display to present content of the evaluation report on a sixth display interface.

25. The device according to any one of claims 17 to 24, wherein the processor is configured to:
control the transceiver to receive breathing detection data when controlling the display to present the second display interface;
compare the breathing detection data with data of the breathing beat image; and
present a reminder or waring to a user when there is a deviation between the breathing detection data and the data of the breathing beat image or the deviation reaches a threshold.

26. The device according to any one of claims 17 to 25, wherein the breathing training time data comprises at least one of time length data of each breath and time length data of the breathing training.

27. The device according to claim 26, wherein a time length of each beat of the breathing beat image is presented by a bubble icon, and
the first display interface presents a plurality of bubble icons which are slidable, and the plurality of bubble icons present different time lengths for a user to choose.

28. The device according to claim 26, wherein the time length data of the breathing training is presented on at least one of the first display interface and the second display interface through a time bar icon, and the time bar icon dynamically presents the time length of the breathing training and presents an effective time length and an optimal time length of the breathing training.

29. A device of providing information for breathing training, comprising a memory, a processor, and a computer program stored in the memory and being executable on the processor, wherein the processor executes the computer program to implement the method according to any one of claims 1 to 16.

30. A system of providing information for breathing training, comprising the device for providing information for breathing training according to claim 29 and a sphygmomanometer, wherein the sphygmomanometer is configured to provide blood pressure data to the device for providing information for breathing training.

31. The system according to claim 30, further comprising:
a breathing detection device, configured to detect breathing data of a user and provide the breathing data to the device for providing information for breathing training.

32. A computer-readable storage medium having a computer program stored thereon that, when being executed by a processor, implements the method according to any one of claims 1 to 16.
